# EUROPÄISCHE PATENTANMELDUNG

(11) **EP 3 586 904 A1**
(43) Veröffentlichungstag der Anmeldung: **01.01.2020**
(21) Anmeldenummer: 19189245.4
(22) Anmeldetag: 14.08.2015
(51) Int. Cl.: A61M 31/00, A61F 2/00, A61M 25/00, A61M 25/02

(54) **ARZNEIMITTELTRÄGER ZUM BEHANDELN UND VORBEUGEN VON KRANKHAFTEN ZUSTÄNDEN IM UROGENITALRAUM**

(30) Priorität: 18.08.2014 DE 102014012206
(62) Teilanmeldung aus: 15774848.4
(71) Anmelder: Inura Medical AG, 6003 Luzern (CH)
(72) Erfinder: Beccioloni, Jean-Jacques, 8954 Geroldswil (CH); von Stetten, Otto, 52072 Aachen (DE)
(74) Vertreter: IPrime Rentsch Kaelin AG

(57) **Zusammenfassung**

Ein Arzneimittelträger (1, 1a), der zum Behandeln und Vorbeugen von krankhaften Zuständen im urogenitalen Raum einer Person, insbesondere im Bereich der Urethra, geeignet ist, besteht aus einem bei Körpertemperatur der Person nicht schmelzbaren biokompatiblen Material, wobei der Arzneimittelträger mit einem Wirkstoff oder einer Wirkstoffmischung zum Behandeln des urogenitalen Raums präpariert ist, wobei sich der Wirkstoff oder die Wirkstoffmischung nicht auflöst oder abgegeben wird.

## Beschreibung

Die Erfindung betrifft einen Arzneimittelträger, der zum Behandeln und Vorbeugen von krankhaften Zuständen im urogenitalen Raum geeignet ist und in eine Körperöffnung einer Person im urogenitalen Raum eingeführt wird, insbesondere in die Urethra, d.h. den Harnleiter. Der Arzneimittelträger kann auch in die Vagina oder sogar den Anus eingeführt werden.

Mit der Erfindung ist ferner eine Methode zur mikrobiellen Sanierung des urogenitalen Raums möglich, eine Methode zur lokalen Applikation von Arzneistoffen im urogenitalen Raum, ferner ein Verfahren zur keimfreien Katheterisierung, insbesondere der Blase, sowie eine Methode zur Verankerung eines Katheters im Körper.

Krankhafte Zustände des Urogenitaltraktes, z.B. bakterielle Infektionen, Nycturie, d.h. vermehrtes, nächtliches Wasserlassen etc. werden häufig mit systemisch applizierten Medikamenten behandelt, die entweder oral oder durch die Haut, d.h. transdermal, oder durch Infusionen von Medikamentenlösungen in die Vene eines Patienten verabreicht werden. Dabei ist es meistens nötig, relativ hohe Dosierungen des Medikaments einzusetzen, um die gewünschte Wirkung zu erzielen. Dies ist dann mit einer entsprechend hohen Rate von unerwünschten Nebenwirkungen verbunden.

Dies könnte bei einer lokalen Applikation der Medikamente weitgehend vermieden werden.

Dazu wurden Suppositorien vorgeschlagen, die in die relativ kurze Urethra einer weiblichen Person eingeführt werden und dort einen Wirkstoff eines Medikamentes freisetzen.

In der US-Patentschrift US 5,085,650 wird ein Suppositorium beschrieben, das einen zylindrischen Körper aufweist, der an einem, dem proximalen Ende, als konischer Anschlag ausgeformt ist, der bei eingeführtem Suppositorium am Eingang der Urethra anliegt und eine Wanderung des Suppositoriums in die Blase verhindert. Am anderen distalen Ende ist eine kugelförmige Verdickung vorgesehen, die bei eingeführtem Suppositorium in der Blase liegt und dadurch das Zurückstossen des Suppositoriums aus der Urethra verhindert. Das Suppositorium besteht aus einem Medikament, das in einen Grundstoff eingebettet ist, der in der Urethra langsam schmilzt und dabei den Wirkstoff freigibt.

Die kugelförmige Verdickung des Suppositoriums kann in der Blase zu Irritationen führen, z.B. Schmerzen und einem zusätzlichen Harndrang.

Ein Suppositorium gemäss der US-Patentschrift US 6,464,670 vermeidet dies. Das Suppositorium weist an dem proximalen Ende einen Anschlag auf, der am Eingang der Urethra anliegt und eine Wanderung des Suppositoriums in Richtung der Blase verhindert. An den Anschlag schliesst sich ein keulenförmiger, in Richtung der Blase konisch sich erweiternder Körper an, der jedoch nicht in Kontakt mit dem enervierten Eingang der Blase gelangt. Der konische Verlauf verhindert ein Ausstossen des Suppositoriums aus der Urethra, da entsprechende muskuläre Haltekräfte auf den keulenförmigen Körper ausgeübt werden.

Die genannten Suppositorien sind aus einem Stoff, z.B. Fetten, Wachsen oder Ölen hergestellt, der bei Körpertemperatur schmilzt. Sie sind nicht sehr formstabil und können beim Platzieren oder während des Schmelzvorganges in der Urethra brechen. Die Bruchstücke können in die Blase gelangen oder ausgestossen werden.

Dies soll gemäss der US-Patentschrift US 7,267,670 vermieden werden, indem das Suppositorium einen Kunststoffkern enthält, der am proximalen Ende mit einer verbreiterten Kunststoffbasis verbunden ist, die beim eingeführten Suppositorium am Eingang der Urethra anliegt. Der Kunststoffkern des Suppositoriums wird nach dem Schmelzen des aufgebrachten Medikaments wieder entfernt. Die Handhabung ist umständlich und schwierig, da sich die Kunststoffbasis nicht gut greifen lässt.

Aber auch bei diesem Design können während des Schmelzvorgangs des Medikaments Bruchstücke auftreten, die in die Blase gelangen können.

Die vorgeschlagenen Suppositorien sind nur zum Einführen in die weibliche Urethra vorgesehen; für die männliche Urethra ist kein Suppositorium zur Behandlung und Vorbeugung von Harnweginfektionen bekannt.

Der Erfindung liegt die Aufgabe zugrunde, einen Arzneimittelträger zum Behandeln und/oder Vorbeugen von krankhaften Zuständen im urogenitalen Raum anzugeben, der während des Einführens in den urogenitalen Raum und auch während der Verweildauer dort stabil bleibt, sodass eine Abgabe eines Wirkstoffs ohne Komplikationen möglich ist.

Diese Aufgabe ist gemäss der Erfindung durch die Merkmale des Patentspruchs 1 gelöst.

Ein Arzneimittelträger besteht gemäss der Erfindung aus einem bei Körpertemperatur der behandelten Person nicht schmelzbaren Material und ist im Wesentlichen stab- oder keulenförmig. Seine Form ähnelt dem eines Suppositoriums, das Material des Arzneimittelträgers ist aber nicht schmelzbar bei der Körpertemperatur der zu behandelnden Person. An seinem proximalen Ende ist vorzugsweise ein Anschlag vorgesehen, der an der Körperöffnung anliegt. Der Arzneimittelträger ist mit einem bei Körpertemperatur nicht schmelzbaren Wirkstoff oder einer Wirkstoffmischung präpariert, z.B. beschichtet.

Der Arzneimittelträger ist so geformt, dass er je mach gewünschter Anwendung z. B. in die Urethra, die Vagina oder sogar in den Anus eingeführt werden kann.

Der Arzneimittelträger weist vorzugsweise eine proximale Basis auf, die bei eingeführtem Arzneimittelträger an dem Eingang der Körperöffnung, insbesondere der Urethra, anliegt und eine Wanderung des Arzneimittelträgers in die Körperöffnung verhindert. An die Basis schliesst sich in distaler Richtung ein Wirkstoffträger an, der stab- oder keulenförmig ausgebildet ist, und bei einer Behandlung der Urethra insbesondere nicht in die Blase der Person reicht.

Die Basis und der Wirkstoffträger bestehen aus einem biokompatiblen, bei Körpertemperatur der Person nicht schmelzbaren Material. Der Wirkstoffträger ist mit einem Wirkstoff oder einer Wirkstoffmischung präpariert, z.B. zumindest teilweise beschichtet.

Durch diese Ausgestaltung kann der Arzneimittelträger einfach in eine Körperöffnung, z.B. die Urethra, und zwar sowohl bei einer weiblichen als auch bei einer männlichen Person eingeführt werden und bleibt dann an dem platzierten Ort.

Der Arzneimittelträger kann nach der Platzierung und nach Abschluss der Behandlung, z.B. durch ein Rückholbändchen wieder entfernt werden.

Der Arzneimittelträger zum Behandeln von krankhaften Zuständen im urogenitalen Raum einer Person, und hier insbesondere im Bereich der Urethra, ist so gestaltet, dass der Blaseneingang der Person nicht berührt wird und keine unangenehmen Reaktionen wie Harndrang etc. ausgelöst werden.

Gemäss einer bevorzugen Ausführungsform der Erfindung ist der Wirkstoffträger mit dem Wirkstoff bzw. einer Wirkstoffmischung bzw. einem Stoff, in den der Wirkstoff oder die Wirkstoffmischung integriert ist, zumindest teilweise beschichtet. Diese Beschichtung kann sich, ohne selbst zu schmelzen, während der Behandlung sukzessive auflösen oder z. B. in Mikroflocken abgegeben werden. Der Wirkstoff bzw. die Wirkstoffmischung kann auch in dem bei Körpertemperatur der zu behandelnden Person nicht schmelzbaren Material des Wirkstoffträgers integriert sein.

Es ist möglich, dass sich die Beschichtung nicht auflöst oder abgegeben wird, und der Wirkstoff bzw. die Wirkstoffmischung allein durch den Kontakt mit dem zu behandelnden Raum wirkt.

Gemäss einer weiteren Ausführungsform der Erfindung besteht der Arzneimittelträger bzw. der Wirkstoffträger aus einem Material, das sich während er Behandlung langsam auflöst oder abgebaut wird, wobei dieses Material selbst nicht bei Körpertemperatur schmilzt. Geeignete Materialien sind Polylactate, Anhydride, Polyester etc.

Insbesondere bei einem Arzneimittelträger, vorzugsweise in Form eines Suppositoriums für eine männliche Person muss darauf geachtet werden, dass das Suppositorium und insbesondere der Wirkstoffträger in gewissen Grenzen flexibel ist, um die Bewegungen de Urethra nicht zu stark zu beeinträchtigen; andererseits muss er starr genug sein, damit er problemlos in den Harnleiter eingeführt werden kann.,

Gemäss einer weiteren Ausführungsform der Erfindung weist der Wirkstoffträger einen in Längsrichtung verlaufenden durchgehenden axialen Kanal auf. Dieser Kanal dient, wie ein herkömmlicher Blasenkatheter, zum einen dazu, bei einer längeren Verweildauer des Arzneimittelträgers in der Urethra noch einen Urinabfluss zu gewährleisten.

Der Kanal ist gemäss der Erfindung so dimensioniert, dass durch den Kanal ein Katheter geschoben werden kann. Der Kanal hat hierfür Dimensionen zwischen 0,3 bis 0,6 Zentimetern, insbesondere 0,35 bis 0,45 Zentimetern.

Dieser Kanal ist ebenfalls mit dem Wirkstoff oder der Wirkstoffmischung präpariert, z.B. beschichtet, um beim Einführen des Katheters Infektionskeime nicht in die Blase zu stossen. Hiermit wird eine keimfreie Katheterisierung erreicht

Ausserdem können über den Katheter lokal wirkende Medikamente in die Körperöffnung, z.B. die Blase, eingeführt werde n. Bei entsprechender Konfiguration des Katheters kann auch der Fortgang der Behandlung beobachtet werden.

Der Katheter kann zudem an dem Arzneimittelträger oder in dem Kanal fixiert werden, sodass weitere Hilfsmittel, etwa ein Ballon, zum Fixieren der Lage des Katheters mit den bekannten Irritationen nicht benötigt werden.

Gemäss einer Ausführungsform der Erfindung wird der Wirkstoffträger aus einer bei Körpertemperatur nicht schmelzbaren Arzneimittelmischung hergestellt, in die zusätzlich ein Mucoadhäsivum eingearbeitet ist, sodass der Wirkstoffträger bis zur vollständigen Abgabe des Wirkstoffes oder der Wirkstoffmischung am vorher bestimmten Ort in dem urogenitalen Raum, insbesondere in der Urethra, anhaftet und dort verbleibt.

Die verwendete Wirkstoffmischung kann auch antimikrobiell wirksame Substanzen enthalten, um eine mikrobielle Sanierung des urogenitalen Raums, insbesondere der Urethra, zu ermöglichen. Der Arzneimittelträger verbleibt hierzu eine längere Zeit am Behandlungsort, wodurch unerwünschte Keime oder Mikroben abgetötet werden. Die antimikrobiellen Substanzen werden freigesetzt oder bleiben am Arzneimittelträger haften. Als Substenzen kommen neben den üblichen antiseptischen Substanzen, wie sie in der Desinfektionsmittelindustrie üblich sind, auch Mischungen aus Schwermetallen in Betracht, wie sie beispielsweise von der Firma Bactigard angeboten erden. Im letzten Fall verbleiben die Wirkstoffe auf dem Arzneimittelträger, wirken allein durch den Kontakt. Mit einem derart behandelten Arzneimittelträger kann der urogenitale Raum, insbesondere die Urethra, mikrobiell saniert werden, sodass Mikroben während der Verweildauer des Arzneimittelträgers in dem urogenitalen Raum abgetötet werden.

In einer besonderen Ausführungsform der Erfindung enthält der Arzneimittelträger antibakterielle Substanzen, im Besonderen Antibiotika wie z.B. Cephalexin oder Levofloxacin etc. zur Behandlung von bakteriellen Harnwegserkrankungen.

In einer weiteren Ausführungsform enthält er antifungale Wirkstoffe wie z.B. Ketoconazol oder Fluconazol etc. zur Behandlung von Pilzinfektionen im urogenitalen Bereich.

In einer weiteren Ausführungsform enthält er antiparasitäre Wirkstoffe wie Azithromycin oder Doxycyclin etc. zur Behandlung sexuell übertragbarer Krankheiten wie z.B. Infektionen durch Chlamydia trachomatis.

In einer weiteren Ausführungsform enthält er antivirale Wirkstoffe wie z.B. Acyclovir etc. zur Behandlung viraler Infektionen im Urogenitalraum.

Ein ggf. beschichteter Arzneimittelträger, insbesondere in der Urethra, wird nach Ende der Behandlungszeit wieder entfernt. Die Dosierung von Wirkstoffen oder Wirkstoffmischungen, insbesondere in einem schmalen therapeutischen Fenster wirken, kann über die Verweilzeit des Arzneimittelträgers im urogenitalen Raum gesteuert werden.

Der Arzneimittelträger bzw. ein durch diesen geschobener Katheter können während der Behandlungszeit etwa durch ein Mucoadhäsivum fixiert werden.

Geeignete biokompatible Materialien für einen Arzneimittelträger sind z.B. Polyurethan, Polyethylen, Polypropylen, Silikon, Viskose, Zellulose, Nylon etc. oder ein anderes natürliches oder synthetisches Material, das sich in die vorgegebene Form des Wirkstoffträgers bringen lässt. Solche Materialien können durch Spritzguss, Giessen, 3-D-Druck, Polymerisation in der Form, Aufschäumen etc. verarbeitet werden.

Für Materialien, die sich im urogenitalen Raum, insbesondere der Urethra, langsam auflösen, kommen Polylactat und Anhydride, Polyester etc. beispielhaft in Frage.

Weitere Ausgestaltungen der Erfindung gehen aus den Unteransprüchen hervor.

Die Erfindung ist in Ausführungsbeispielen anhand der Zeichnung näher beschrieben.

In der Zeichnung stellen dar:
**Figuren 1A**, **1B**, **1C und 1D****:** schematische Darstellungen jeweils eines Arzneimittelträgers gemäss der Erfindung in Form eines Suppositoriums zum Einführen in die Urethra einer weiblichen Person;
**Figur 2****:** eine schematische Darstellung eines Arzneimittelträgers gemäss der Erfindung zum Einführen in die Urethra einer männlichen Person;
**Figuren 3A, 3B und 3C****:** schematische Darstellungen von Arzneimittelträgern in Form von Suppositorien zum Einführen in die Urethra einer weiblichen Person (Figur 3A, Figur 3B) bzw. einer männlichen Person (Figur 3C), jeweils mit einem durchgehenden axialen Kanal;
**Figuren 4A und 4B****:** schematische Darstellungen jeweils eines Arzneimittelträgers in Form eines Suppositoriums zum Einführen in die Urethra einer weiblichen Person, mit einer vollständigen bzw. teilweisen Beschichtung mit einem Wirkstoff bzw. einer Wirkstoffmischung;
**Figuren 5A und 5B****:** schematische Darstellungen jeweils eines Arzneimittelträgers zum Einführen in die Urethra einer männlichen Person, mit einem axialen durchgehenden, vollständig mit einem Wirkstoff bzw. einer Wirkstoffmischung beschichteten Kanal (Figur 5A) bzw. einer teilweisen Beschichtung (Figur 5B);
**Figuren 6A und 6B****:** schematische Darstellungen jeweils eines Arzneimittelträgers in Form eines Tampons zum Einführen in die Urethra, wobei der Tampon ausgelegt ist, um länger innerhalb der Urethra zu verbleiben.

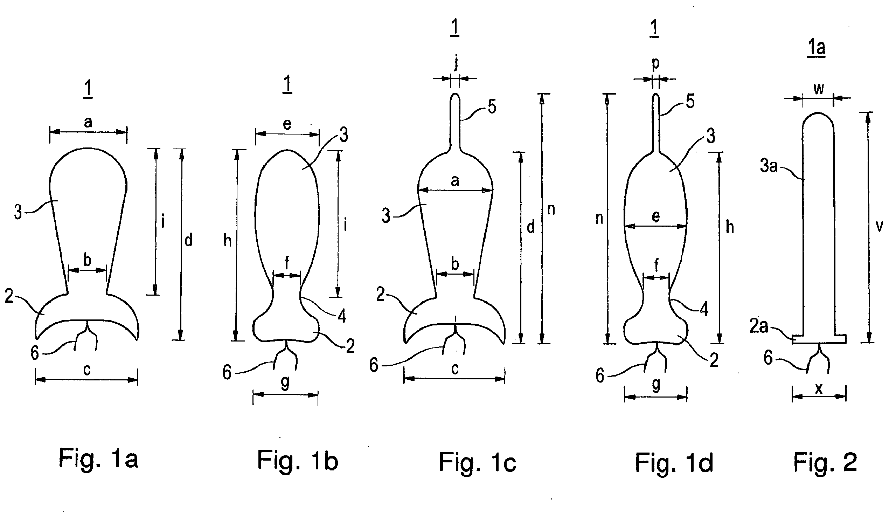

In den Figuren 1A, 1B, 1C und 1D ist jeweils ein Arzneimittelträger 1 in Form eines Suppositoriums dargestellt, der in die Urethra einer weiblichen Person eingeführt wird. Der Arzneimittelträger 1 weist an seinem proximalen Ende eine Basis 2 auf, die bei eingeführtem Arzneimittelträger an dem Eingang der nicht dargestellten Urethra anliegt und diesen abdeckt. An die Basis 2 schliesst sich in distaler Richtung ein im Wesentlichen keulenförmiger Wirkstoffträger 3 an.

Gemäss Figur 1A erweitert sich der keulenförmige Wirkstoffträger 3 ausgehend von der Basis 2 konisch nach distal und ist an seinem distalen Ende abgerundet. Die gesamte Länge **d** des Arzneimittelträgers ist so ausgelegt, dass dieser nicht in die Blase der zu behandelnden Person reicht und kurz vor dem Blaseneingang endet, um Irritationen zu vermeiden.

Der Arzneimittelträger 1 gemäss Figur 1A hat eine Länge **d** von 3,5 bis 4,5 Zentimetern (cm), vorzugsweise 4,0 bis 4,5 cm, und weist am distalen Ende einen Durchmesser **a** von 0,8 bis 1,5 cm auf, vorzugsweise 0,8 bis 1,2 cm. An der proximalen Seite an der Basis 2 hat der Wirkstoffträger 3 einen Durchmesser **b** von 0,4 bis 1,0 cm, vorzugsweise 0,6 bis 0,8 cm. Die Basis 2, die als Anschlag zum Abdecken des Eingangs der Urethra dient, hat einen Durchmesser **c** zwischen 1,0 und 2,0 cm, vorzugsweise 1,3 bis 1,7 cm.

Die angegebenen Masse können je nach der Körperstruktur der zu behandelnden Person variieren. Die Bezugszeichen zu den einzelnen Massen gelten für alle Figuren.

In Figur 1B ist ein Arzneimittelträger 1 gezeigt, der eine Form aufweist, die einem Spielkegel ähnelt. Der Arzneimittelträger 1 weist wiederum eine Basis 2 und einen sich nach distal erstreckenden Wirkstoffträger 3 auf. Er weist von der Basis 2 ausgehend einen sich zunächst verjüngenden Bereich 4 auf, der sich erweitert und kontinuierlich in den keulenförmigen Wirkstoffträger 3 übergeht. Die Breite **g** der Basis 2 beträgt 0,8 bis 1,5 cm, vorzugsweise 0,8 bis 1,2 cm; der Bereich 4 hat in seinem engsten Bereich einen Durchmesser **f** von etwa 0,4 bis 1,0 cm, vorzugsweise 0,6 bis 0,8 cm. Die grösste Breite **e** des Arzneimittelträgers 1 im oberen distalen Bereich des Wirkstoffträgers 3 ist ähnlich der Breite **g** der Basis 2, d.h. 0,8 bis 1,5 cm. Die gesamte Länge **h** des Arzneimittelträgers 1 beträgt etwa 4,0 bis 4,5 cm; von der distalen Spitze bis zur Verjüngung sind es etwa 2,5 bis 3,5 cm, vorzugsweise 2,8 bis 3,2 cm (Länge **i**).

In Figur 1C ist ein Arzneimittelträger 1 ähnlich der Figur 1A gezeigt. Zusätzlich ist jedoch an dem distalen Ende eine dünne axiale Spitze 5 vorgesehen, die in die Blase hineinragt und ebenfalls mit einem Wirkstoff bzw. einer Wirkstoffmischung präpariert ist. Mit dieser Ausbildung des Arzneimittelträgers können mit Hilfe der präparierten Spitze auch krankhafte Zustände der Blase lokal behandelt werden, ohne dass Irritationen auftreten.

Der Durchmesser **j** der Spitze 5 beträgt etwa 0,1 bis 0,3 cm, vorzugsweise 0,15 bis 0,25 cm, deren Länge etwa 1,5 bis 2,5 cm die Gesamtlänge **n** des Arzneimittelträgers mit der Spitze 5 beträgt etwa 5,5 bis 6,5 cm, vorzugsweise 5,0 bis 5,5 cm.

In der Figur 1D ist ein Arzneimittelträger 1 gezeigt, der ähnlich wie in Figur 1B ausgestaltet ist, d.h. mit einer Basis 2 mit einem Durchmesser **g** von 1,0 bis 2,0 cm, an die sich eine Verjüngung 4 und ein keulenförmiger Wirkstoffträger 3 mit einem maximalen Durchmesser **e** von 0,8 bis 1,5 cmanschliesst. Der Arzneimittelträger 1 ist ähnlich wie in Figur 1C mit einer Spitze 5 von etwa 1 bis 2 cm versehen und hat eine Gesamtlänge **n** von etwa 5,5 bis 6,5 cm.

In Figur 2 ist ein Arzneimittelträger 1a zum Einführen in eine männliche Urethra gezeigt. Er weist eine kreisförmige Basis 2a auf, die als Anschlag dient und bei eingeführtem Suppositorium am Eingang der Urethra anliegt. Der Wirkstoffträger 3a ist zylinderförmig. Die Gesamtlänge **v** des Arzneimittelträgers ist 5,0 bis 7,0 cm, vorzugsweise 5,5 bis 6,6 cm; der Durchmesser **w** des Wirkstoffträgers 3a ist 0,4 bis 1,0 cm, vorzugsweise 0,6 bis 0,8 cm. Der Durchmesser **x** der Basis 2a ist 1,2 bis 2,0 cm, vorzugsweise 1,3 bis 1,7 cm.

Um die Arzneimittelträger nach der Behandlungszeit einfacher aus der Urethra entfernen zu können, ist jeweils ein Rückholbändchen 6 mit dem Arzneimittelträger verbunden. Dies ist bei allen Darstellungen in den Figuren angedeutet.

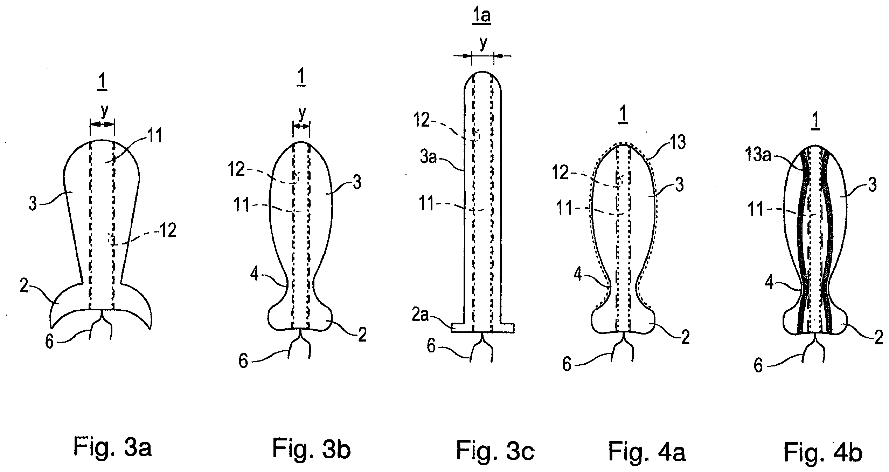

In den Figuren 3A, 3B sowie 4A und 4B sind Arzneimittelträger 1 ähnlich den Figuren 1A bzw. 1B gezeigt, in Figur 3C ein Arzneimittelträger 1a ähnlich der Figur 2.

Die Arzneimittelträger sind jeweils um einen zentralen axialen durchgehenden Kanal 11 ergänzt. Dieser Kanal 11 dient u.a. dazu, einen Urinabfluss zu ermöglichen, auch wenn der Arzneimittelträger über längere Zeit in der Urethra verbleibt. Ausserdem kann durch diesen Kanal 11 bei entsprechenden Dimensionen ein Katheter geschoben werden, der bis in die Blase reicht, um dort ggf. lokal noch Medikamente zu applizieren oder den Behandlungsvorgang durch einen optischen Sensor zu beobachten.

Um zu verhindern, dass mit dem Katheter Mikroben in die Blase geschoben werden, die Blasenentzündungen oder gar Nierenbeckenentzündungen verursachen können, ist der Kanal 11 jeweils mit einer antimikrobiellen Beschichtung 12 versehen. So können bei der Katheterisierung und auch während der Verweildauer des Katheters keine Mikroben in die Blase oder Nieren vordringen.

Der Durchmesser **y** des Kanals 11 liegt bei etwa 0,3 bis 0,6 cm, insbesondere bei 0,35 bis 0,45 cm.

In den Figuren 4A und 4B sind Arzneimittelträger 1 gezeigt, die jeweils in die weibliche Urethra eingeführt werden, wobei in Figur 4A der Wirkstoffträger 3 durchgehend mit einer Beschichtung 13 des Wirkstoffs bzw. der Wirkstoffmischung versehen ist. Diese Beschichtung 13 ist sehr dünn und kann sich während der Verweildauer des Arzneimittelträgers in der Urethra langsam auflösen.

Es ist auch möglich, diese Beschichtung 13 als Kontaktmedikament auszubilden, sodass die Beschichtung 13 während der gesamten Verweildauer in der Urethra verbleibt und der Wirkstoff allein durch den Kontakt mit dem Gewebe der Urethra wirkt.

Dies gilt für alle gezeigten Ausführungsformen, auch wenn dieses nicht dargestellt ist.

In Figur 4B ist ein Arzneimittelträger 1 gezeigt, der anstelle einer vollständigen Beschichtung des Wirkstoffträgers 3 eine Beschichtung 13a nur an einzelnen Stellen, in diesem Falle in Streifen aufweist.

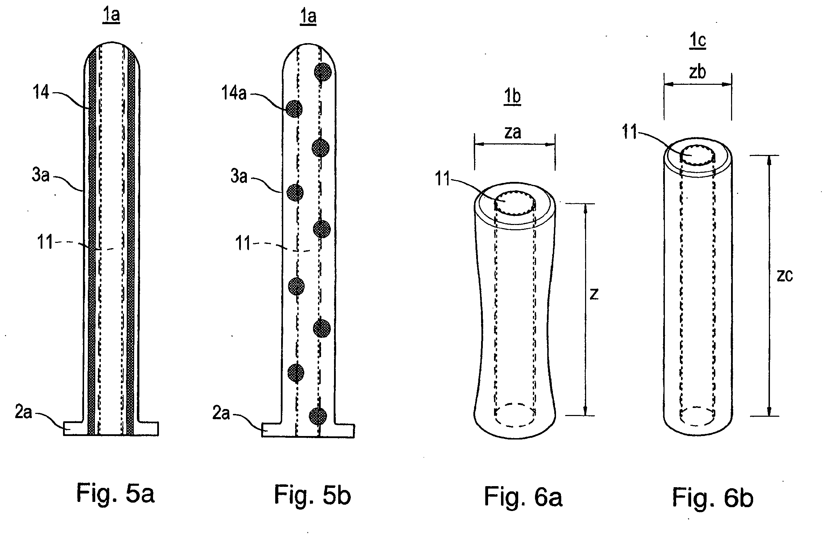

Die Figuren 5A und 5B zeigen jeweils einen Arzneimittelträger 1a zum Einführen in die Urethra einer männlichen Person, wobei der Arzneimittelträger 1a im Wesentlichen aufgebaut ist wie in Figur 2, d.h. eine kreisförmige Basis 2a und einen zylinderförmigen Wirkstoffträger 3a sowie eine durchgehenden Kanal 11 aufweist.

Um die gezeigten Arzneimittelträger in der weiblichen oder männlichen Urethra besser zu halten, können die Arzneimittelträger mit mucoadhäsiven Substanzen beschichtet sein, wobei die Beschichtung z.B. in Streifen 14 gemäss Figur 5A bzw. punktuell 14a aufgebracht ist, wie in Figur 5b gezeigt.

Solche Materialien sind z.B. Carbomere, Poloxamere, Polyacrylate und Derivative, Chitosane und Chitosanderivate, Alginatpolyethylenglycolacrylate, Thiomere (z. B. Polycarbophilcystein etc.), Lectine sowie Mischungen aus den genannten Stoffen. Die Menge des Mucoadhäsivums wird dabei so gewählt, dass der Arzneimittelträger nach der gewünschten Verweildauer schmerzfrei entfernt werden kann.

In den Figuren 6A und 6B sind Arzneimittelträger 1b bzw. 1c dargestellt, die in ihrer Form einem handelsüblichen Tampon mit geringem Durchmesser ähneln. Die Arzneimittelträger sind hierbei aus einer bei Körpertemperatur nicht schmelzbaren Arzneimittelmischung hergestellt, in die zusätzlich ein Mucoadhäsivum eingearbeitet oder mit einem solchen beschichtet ist, sodass die Arzneimittelträger bis zum Ende der Behandlung am vorher bestimmten Ort im Urogenitalraum, insbesondere der Urethra, verbleiben. Der Arzneimittelträger 1b gemäss Figur 6A hat eine Länge **z** von z.B. 2,5 bis 3,5 cm und einen Durchmesser **za** von 0,8 bis 1,5 cm. Dieser Arzneimittelträger 1b kann wiederum einen durchgehenden Kanal 11 aufweisen. Der Arzneimittelträger 1c gemäss Figur 6b weist eine Länge **zc** von ca.5,0 bis 7,0 cm auf, der Durchmesser **zb** beträgt etwa 0,4 bis 1,0 cm. Auch bei dieser Ausführung kann jeweils ein durchgehender Kanal 11 für die oben genannten Zwecke vorgesehen sein.

Alle beschrieben Arzneimittelträger können manuell oder mit einem Applikator platziert werden.

Methoden für die Beschichtung des Arzneimittelträgers mit einem durchgehenden Überzug, mit einem teilweisen Überzug, z.B. in Streifen oder an einzelnen Punkten, sind in der pharmazeutischen Wissenschaft bekannt, z.B. Tauchen, Aufschmelzen, Besprühen, Gefriertrocknen, etc. Gegebenenfalls werden die Arzneimittelträger mit Plasma vorbehandelt, um eine bessere Haftung zu erzielen. Die jeweiligen Wirkstoffe und Dosierungen ergeben sich aus den entsprechenden Therapien.

Der mit einem Wirkstoff präparierte bzw. beschichtete Arzneimittelträger wird in den Urogenitalraum eingeführt und nach einer vorbestimmten Zeit mit dem Rückholbändchen 6 wieder entfernt. Da die Freisetzungskinetik der jeweiligen Arzneimittelmischungen bekannt ist bzw. vorher bestimmt worden ist, kann über die Verweildauer des Arzneimittelträgers im urogenitalen Raum die Dosis der Arzneimittelmischengen exakt eingestellt werden. So gelingt es auch, Wirkstoffe mit einem schmalen therapeutischen Fenster, bei denen der Abstand zwischen wirksamer und toxischer Arzneimittelmischung gering ist, zielgenau zu dosieren.

Aus dem Vorhergehenden ist verständlich, dass ein präparierter Arzneimittelträger gemäss der Erfindung generell zur Applikation eines Wirkstoffs oder einer Wirkstoffmischung oder anderer, etwa antimikrobieller Substanzen verwendet werden kann, um krankhafte Zustände des urogenitalen Raums zu behandeln oder diesen zu sanieren.

Nachfolgend wird eine Reihe vorteilhafter Ausführungsformen von Arzneimittelträgern beschrieben.

Ein vorteilhafter Arzneimittelträger, der zum Behandeln und Vorbeugen von krankhaften Zuständen im urogenitalen Raum einer Person, insbesondere im Bereich der Urethra, geeignet ist, besteht aus einem bei Körpertemperatur der Person nicht schmelzbaren biokompatiblen Material, wobei der Arzneimittelträger mit einem Wirkstoff oder einer Wirkstoffmischung zum Behandeln des urogenitalen Raums präpariert ist.

Vorteilhaft ist der Arzneimittelträger mit einem bei Körpertemperatur der Person nicht schmelzbaren Wirkstoff bzw. einer Wirkstoffmischung präpariert.

Alternativ oder zusätzlich kann der Arzneimittelträger mit dem Wirkstoff bzw. der Wirkstoffmischung zumindest teilweise beschichtet sein.

Alternativ oder zusätzlich kann der Arzneimittelträger zum Einführen in eine Körperöffnung im urogenitalen Raum stab- oder keulenförmig ausgebildet sein.

Alternativ oder zusätzlich kann der Arzneimittelträger zum Einführen in eine Körperöffnung, insbesondere die Urethra, eine proximale Basis, die als Anschlag zum Abdecken der Körperöffnung dient, und einen sich nach distal anschliessenden stab- oder keulenförmigen Wirkstoffträger aufweist, der mit dem Wirkstoff bzw. der Wirkstoffmischung präpariert ist.

Borteilhaft ist bei einer solchen Ausführungsform eines Arzneimittelträgers der Wirkstoff oder die Wirkstoffmischung in dem Material des Wirkstoffträg integriert.

Alternativ oder zusätzlich kann bei einem der vorgenannten Arzneimittelträger der Wirkstoff oder die Wirkstoffmischung in einem Material vorliegen, das bei Körpertemperatur der Person sich langsam auflöst.

Alternativ oder zusätzlich kann der Arzneimittelträger einen durchgehenden axialen Kanal aufweisen.

Vorteilhaft ist bei einer solchen Ausführungsform eines Arzneimittelträgers der Kanal so dimensioniert, dass ein Katheter durchgeschoben werden kann.

In einer besonders vorteilhaften Ausführungsform eines solchen Arzneimittelträgers ist der Katheter in dem Kanal fixierbar.

Bei den vorgenannten vorteilhaften Ausführungsformen eines solchen Arzneimittelträgers ist vorteilhaft der Kanal mit dem Wirkstoff oder der Wirkstoffmischung und/oder einer antimikrobiellen Substanz präpariert.

Alternativ oder zusätzlich kann der Arzneimittelträger am distalen Ende eine axiale Spitze aufweisen, die mit dem Wirkstoff oder der Wirkstoffmischung präpariert ist.

Alternativ oder zusätzlich kann der Arzneimittelträger anschliessend an eine Basis einen sich verjüngenden Bereich aufweisen, der sich erweitert und in die darauffolgende stab- oder keulenförmige Kontur des Arzneimittelträgers übergeht.

Alternativ oder zusätzlich kann der Arzneimittelträger im Wesentlichen vollständig oder in Streifen oder an einzelnen Punkten mit dem Wirkstoff oder der Wirkstoffmischung beschichtet sein.

Vorteilhaft ist bei einer solchen Ausführungsform eines Arzneimittelträgers zur Fixierung des Arzneimittelträgers in der Körperöffnung dieser mit mucoadhäsiven Substanzen versehen ist, die gegebenenfalls in dem Wirkstoff oder der Wirkstoffmischung integriert sind.

Vorteilhaft ist bei einer solchen Ausführungsform eines Arzneimittelträgers die Menge der mucoadhäsiven Substanzen so gewählt, dass der Arzneimittelträger nach einer vorbestimmten Zeit schmerzfrei entfernt werden kann.

Alternativ oder zusätzlich kann der Arzneimittelträger mit antimikrobiellen Substanzen präpariert sein, die während der Verweildauer des Arzneimittelträgers in der Körperöffnung freigesetzt werden oder am Arzneimittelträger fest haften.

Vorteilhaft ist bei einer solchen Ausführungsform eines Arzneimittelträgers der Arzneimittelträger in die Urethra einer Person einführbar und verbleibt dort eine längere Zeit, sodass unerwünschte Keime abgetötet und Infektionen verhindert werden.

Alternativ oder zusätzlich kann der Arzneimittelträger aus einem biokompatiblen Grundstoff bestehen, der sich im urogenitalen Raum langsam auflöst, z.B. Polylactat, Anhydride, Polyester etc.

Alternativ oder zusätzlich ist bei einem Arzneimittelträger die Dosierung des Wirkstoffs bzw. der Wirkstoffmischung über die Verweilzeit des Arzneimittelträgers in der Körperöffnung steuerbar.

Alternativ oder zusätzlich kann der Arzneimittelträger antibakterielle Substanzen, im Besonderen Antibiotika wie z.B. Cephalexin oder Levofloxacin etc. zur Behandlung von bakteriellen Harnwegserkrankungen enthalten.

Alternativ oder zusätzlich kann der Arzneimittelträger antifungale Wirkstoffe wie z.B. Ketoconazol oder Arzneimittelträger nach einem der vorhergehenden Ansprüche, dadurch gekennzeichnet, dass Fluconazol etc. zur Behandlung von Pilzinfektionen im urogenitalen Bereich enthalten.

Alternativ oder zusätzlich kann der Arzneimittelträger antiparasitäre Wirkstoffe wie Azithromycin oder Doxycyclin etc. zur Behandlung sexuell übertragbarer Krankheiten wie z.B. Infektionen durch Chlamydia trachomatis enthalten.

Alternativ oder zusätzlich kann der Arzneimittelträger antivirale Wirkstoffe wie z.B. Acyclovir etc. zur Behandlung viraler Infektionen im Urogenitalraum enthalten.

Alternativ oder zusätzlich kann der Arzneimittelträger zum Entfernen aus der Körperöffnung mit einem Rückholbändchen ausgestattet sein.

Nachfolgend wird eine Reihe vorteilhafter Verwendungen der vorgenannten vorteilhaften Arzneimittelträger beschrieben.

Bei einer vorteilhaften Verwendung eines vorgenannten vorteilhaften Arzneimittelträgers wird zur Applikation eines Wirkstoffs, einer Wirkstoffmischung und/oder einer anderen Substanz in dem urogenitalen Raum einer Person der damit präparierte Arzneimittelträger in den urogenitalen Raum eingeführt und nach einer bestimmten Zeit entfernt.

Bei einer anderen vorteilhaften Verwendung eines vorgenannten vorteilhaften Arzneimittelträgers wird zur mikrobiellen Sanierung des Urogenitaltrakts, insbesondere der Urethra, ein mit antimikrobiellen Substanzen präparierter Arzneimittelträger in eine Körperöffnung des Urogenitaltrakts, insbesondere die Urethra, eingeführt und verbleibt dort eine längere Zeit, um unerwünschte Keime abzutöten und Infekte zu verhindern.

Bei einer weiteren vorteilhaften Verwendung eines der vorgenannten vorteilhaften Arzneimittelträger mit Kanal wird zur keimfreien Katheterisierung der durchgehende Kanal des Arzneimittelträgers, der zur Aufnahme eines Katheters ausgelegt ist, mit antimikrobiellen Substanzen präpariert.

## Patentansprüche

1. Arzneimittelträger, der zum Behandeln und Vorbeugen von krankhaften Zuständen im urogenitalen Raum einer Person, insbesondere im Bereich der Urethra, geeignet ist, bestehend aus einem bei Körpertemperatur der Person nicht schmelzbaren biokompatiblen Material, wobei der Arzneimittelträger mit einem Wirkstoff oder einer Wirkstoffmischung zum Behandeln des urogenitalen Raums präpariert ist, wobei sich der Wirkstoff oder die Wirkstoffmischung nicht auflöst oder abgegeben wird.

2. Arzneimittelträger nach Anspruch 1, wobei der Arzneimittelträger mit einem bei Körpertemperatur der Person nicht schmelzbaren Wirkstoff bzw. einer Wirkstoffmischung präpariert ist.

3. Arzneimittelträger nach einem der vorhergehenden Ansprüche, wobei der Arzneimittelträger mit dem Wirkstoff bzw. der Wirkstoffmischung zumindest teilweise beschichtet ist.

4. Arzneimittelträger nach einem der vorhergehenden Ansprüche, wobei der Arzneimittelträger zum Einführen in eine Körperöffnung im urogenitalen Raum stabförmig oder keulenförmig ausgebildet ist.

5. Arzneimittelträger nach einem der vorhergehenden Ansprüche, wobei der Arzneimittelträger zum Einführen in eine Körperöffnung, insbesondere die Urethra, eine proximale Basis aufweist, die als Anschlag zum Abdecken der Körperöffnung dient, und einen sich nach distal anschliessenden stabförmigen oder keulenförmigen Wirkstoffträger aufweist, wobei der Wirkstoffträger mit dem Wirkstoff bzw. der Wirkstoffmischung präpariert ist, und/oder wobei bei dem Wirkstoffträger der Wirkstoff oder die Wirkstoffmischung in dem Material des Wirkstoffträgers integriert ist.

6. Arzneimittelträger nach einem der vorhergehenden Ansprüche, wobei der Arzneimittelträger einen durchgehenden axialen Kanal aufweist; oder
der Arzneimittelträger einen durchgehenden axialen Kanal aufweist, welcher so dimensioniert ist, dass ein Katheter durchgeschoben werden kann; oder
der Arzneimittelträger einen durchgehenden axialen Kanal aufweist, welcher so dimensioniert ist, dass ein Katheter durchgeschoben werden kann, wobei der Katheter in dem Kanal fixierbar ist.

7. Arzneimittelträger nach Anspruch 6, wobei der Kanal mit dem Wirkstoff oder der Wirkstoffmischung und/oder einer antimikrobiellen Substanz präpariert ist.

8. Arzneimittelträger nach einem der vorhergehenden Ansprüche, wobei der Arzneimittelträger am distalen Ende eine axiale Spitze aufweist, die mit dem Wirkstoff oder der Wirkstoffmischung präpariert ist.

9. Arzneimittelträger nach einem der vorhergehenden Ansprüche, wobei der Arzneimittelträger im Wesentlichen vollständig oder in Streifen oder an einzelnen Punkten mit dem Wirkstoff oder der Wirkstoffmischung beschichtet ist; oder wobei der Arzneimittelträger im Wesentlichen vollständig oder in Streifen oder an einzelnen Punkten mit dem Wirkstoff oder der Wirkstoffmischung beschichtet ist und zur Fixierung des Arzneimittelträgers in der Körperöffnung dieser mit mucoadhäsiven Substanzen versehen ist, die gegebenenfalls in dem Wirkstoff oder der Wirkstoffmischung integriert sind, wobei optional die Menge der mucoadhäsiven Substanzen so gewählt ist, dass der Arzneimittelträger nach einer vorbestimmten Zeit schmerzfrei entfernt werden kann.

10. Arzneimittelträger nach einem der vorhergehenden Ansprüche, wobei der Arzneimittelträger mit antimikrobiellen Substanzen präpariert ist; oder wobei der Arzneimittelträger mit antimikrobiellen Substanzen präpariert ist und in die Urethra einer Person einführbar ist und dort eine längere Zeit verbleibt, sodass unerwünschte Keime abgetötet und Infektionen verhindert werden.

11. Arzneimittelträger nach einem der vorhergehenden Ansprüche, wobei die Dosierung des Wirkstoffs bzw. der Wirkstoffmischung über die Verweilzeit des Arzneimittelträgers in der Körperöffnung steuerbar ist.

12. Arzneimittelträger nach einem der vorhergehenden Ansprüche, wobei der Arzneimittelträger
a) antibakterielle Substanzen, im Besonderen Antibiotika wie z.B. Cephalexin oder Levofloxacin etc. zur Behandlung von bakteriellen Harnwegserkrankungen; und/oder
b) antifungale Wirkstoffe wie z.B. Ketoconazol oder Fluconazol etc. zur Behandlung von Pilzinfektionen im urogenitalen Bereich; und/oder
c) antiparasitäre Wirkstoffe wie Azithromycin oder Doxycyclin etc. zur Behandlung sexuell übertragbarer Krankheiten wie z.B. Infektionen durch Chlamydia trachomatis; und/oder
d) antivirale Wirkstoffe wie z.B. Acyclovir etc. zur Behandlung viraler Infektionen im Urogenitalraum
enthält.

13. Arzneimittelträger nach einem der vorhergehenden Ansprüche, wobei der Arzneimittelträger zum Entfernen aus der Körperöffnung mit einem Rückholbändchen ausgestattet ist.

14. Arzneimittelträger nach einem der vorhergehenden Ansprüche zur Verwendung in der
a) Applikation eines Wirkstoffs, einer Wirkstoffmischung und/oder einer anderen Substanz in dem urogenitalen Raum einer Person, wobei der Arzneimittelträger in den urogenitalen Raum eingeführt und nach einer bestimmten Zeit entfernt wird; oder
b) mikrobiellen Sanierung des Urogenitaltrakts, insbesondere der Urethra, wobei ein mit antimikrobiellen Substanzen präparierter Arzneimittelträger in eine Körperöffnung des Urogenitaltrakts, insbesondere die Urethra, eingeführt wird und dort eine längere Zeit verbleibt, um unerwünschte Keime abzutöten und Infekte zu verhindern.

15. Arzneimittelträger nach Anspruch 7 zur Verwendung in der keimfreien Katheterisierung, wobei der durchgehende Kanal des Arzneimittelträgers, der zur Aufnahme eines Katheters ausgelegt ist, mit antimikrobiellen Substanzen präpariert ist.
